# EUROPEAN PATENT SPECIFICATION

(11) **EP 3 150 173 B1**
(45) Date of publication and mention of the grant of the patent: **28.02.2018**
(21) Application number: 16382452.7
(22) Date of filing: 03.10.2016
(51) Int. Cl.: A61F 2/24, A61B 17/06

(54) **REABSORBABLE SUBAORTIC RING**
RESORBIERBARER SUBAORTISCHER RING
ANNEAU SOUS-AORTIQUE RÉSORBABLE

(30) Priority: 02.10.2015 ES 201531420
(43) Date of publication of application: 05.04.2017
(73) Proprietor: Aramendi Gallardo, José Ignacio, 48200 Durango (Bizkaia) (ES)
(72) Inventor: Aramendi Gallardo, José Ignacio, 48200 Durango (Bizkaia) (ES)
(74) Representative: Pons Ariño, Angel

(56) References cited:
- EP-A1- 0 338 994
- WO-A1-01/89426
- WO-A2-2012/064902
- US-A1- 2005 197 696
- US-B1- 6 416 549

## Description

### OBJECT OF THE INVENTION

This invention refers to a resorbable subaortic ring for repairing aortic valve insufficiency.

### BACKGROUND OF THE INVENTION

Severe valvular insufficiency is a disease of the valves regulating blood flow in the heart that prevents the valves from closing fully, allowing a return blood flow in the opposite direction to physiological normal, which results in progressive pathological injury. Of the various heart valves, the aortic sigmoid valve, located between the left ventricle and the aortic artery, prevents blood returning from the aorta to the left ventricle. It consists of three membranes, two anterior and one posterior, and has a morphology similar to that of a swallow nest. Insufficiency causes an abnormal flow of blood in diastole from the aorta toward the left ventricle, which requires repair techniques at various levels, among which are actions on the valvular ring.

This ring of the aortic valve is not circular but is similar to a crown with three peaks, coinciding with the areas where the corners meet. The expansion of this ring, responsible for malfunction of the valve, is repaired by an operation known as annuloplasty, consisting of a reduction of the annular diameter to that of the bodily size, which in an adult measures some 26 millimetres. This operation enables better coaptation of the leaflets and helps the repair to be long lasting and the native ring, that is the natural existing ring, to not gradually dilate again.

Currently, there are two techniques for performing aortic annuloplasty:
- Schäfers' "plication stitch", consisting of applying a subaortic circular suture with a Gore-Tex® suture that is internally or externally implanted immediately below the native ring. Unlike this native ring, it does not need to follow the crown shape but is circular in a strictly horizontal plane. The stitch is tied by inserting a Hegar dilator, which is a 26 millimetre cylinder, like a mould so that the suture remains exactly at this diameter. This is a skilled and imperfect technique, subject to variability of the surgeon, and the repair also is rigid, not allowing for the changes that occur in the ring over the cardiac cycle, as in systole the diameter is increased to encourage the flow of blood and in diastole the nominal diameter is reduced.
- Coroneo external ring, a polyethylene terephthalate ring with a core of very flexible silicone, which is inserted outside the aorta with 6 fixing stitches. It allows diameter changes occurring during the cardiac cycle and comes in various sizes from 25 to 29 millimetres. It demands meticulous dissection of the root of the heart and forces resection and reimplantation of the coronary arteries, so it is limited to the more complex cases that require replacement of the aortic root with preservation of the valve; it is known as the Yacoub operation.

Document WO0189426 A1 discloses a device for shrinking and/or reinforcing the heart valve annulus including a thick link made of a bioresorbable, soft and curved material having at both ends thereof a thin yarn with a curved needle.

### DESCRIPTION OF THE INVENTION

The object of the invention is a cardiac prosthetic medical device and refers to an internal subaortic ring, as disclosed in the appended claims, in the shape of an open circle to facilitate its implantation in an annuloplasty operation. It is made of a flexible material and resorbable by the human body such as, for example, a polydioxanone polymer, that resolves the problems and drawback created by the previously described annuloplasty techniques.

Said ring has an open circular shape to facilitate its placement in the valve of the patient, and it is closed at the time of tying, thereby achieving a suitable valvular diameter of some 26 millimetres for the case of a standard adult.

The ring is a tubular structure, preferably with a diameter of 1.5 millimetres, made of a flexible material and resorbable after a period of approximately one year after surgery such as, for example, a polydioxanone polymer, that also allows for the usual fluctuations that occur in the native ring both in systole and in diastole. The ring is preferably made in two sizes, specifically 26 and 28 millimetres in diameter.

This subaortic ring is surgically implanted inside the aortic valve in a horizontal circular way such as by Schäfers' stitch technique, embedding it in the myocardium just below the native aortic ring. Thus it can be implanted in all cases of aortic repair, without the need to dissect all the aortic root nor to section and re-implant the coronaries, except if it is necessary to perform the previously mentioned Yacoub operation.

In more detail, the implantation of this device is performed by an "open heart" cardiac operation for repairing aortic valvular insufficiency. When the extra-corporeal circulation has been connected, the ascending aorta is opened transversally and the aortic valve is exposed. At this time, the valve is examined and the leaflets analysed, checking that there is sufficient tissue and identifying the cause of the insufficiency, which is generally due to the prolapse of a leaflet. Depending on the information obtained and if the surgeon decides that the valve can be repaired, the native ring is measured using the Hegar dilator, checking that it is effectively dilated, which occurs when its average diameter is over 25 millimetres. If this dilation measures between 25 and 28 millimetres, the 26 millimetre prosthetic valvular ring must be implanted, whereas if the dilation is equal to or greater than 28 millimetres, the 28 millimetres ring will be necessary.

When the suitable ring has been chosen, it is opened and implanted, suturing the extension with a single filament suture from one end, starting below the internal valvular plane at the level of the corner between the non-coronary leaflet and the right coronary, to later continue applying stitches in a clockwise direction, progressively burying the prosthetic ring in the myocardium, just below the valvular plane.

This is continued until returning to the previously mentioned corner, where the membranous septum is found, which must not be surrounded by the suture to prevent an atrioventricular block or AV block. To do this, the two needles at the ends of the ring are passed from inside to outside the left ventricle, toward the external space between the ascending aorta and the left atrial ceiling, allowing the needles to pass on both sides of the membranous septum and thus preventing the AV block.

Both sutures are tied outside the heart on a Teflon pad that closes the circle, creating an effective subaortic annuloplasty of the desired size. From this point, the aortic repair continues according to the usual technique, normally by central stitches in the leaflet until reaching an appropriate height of the leaflet. The final quality of the intervention is checked by transoesophageal echocardiographic monitoring.

The resorbable subaortic ring thus described surgically corrects the dilation of the aortic valvular ring, thus preventing the pathological problems caused by this dilation.

Some of the more noteworthy benefits are that it is not thrombogenic, that is, does not constitute a factor that could cause a thrombus or clot. Also, as it is not buried within the myocardial ring, it never enters into contact with the blood flow, so does not require anticoagulant or antiplatelet treatment. For this same reason, it is resistant to infections and can be used in cases of endocarditis. Finally, it is totally biocompatible and is not rejected.

### DESCRIPTION OF THE FIGURES

To complement the description being made and with the aim of helping better understanding of the characteristics of the invention in accordance with a preferred embodiment, a set of figures is attached to this description that forms an integral part of thereof, wherein for the purposes of illustration and without limitation the following are shown:

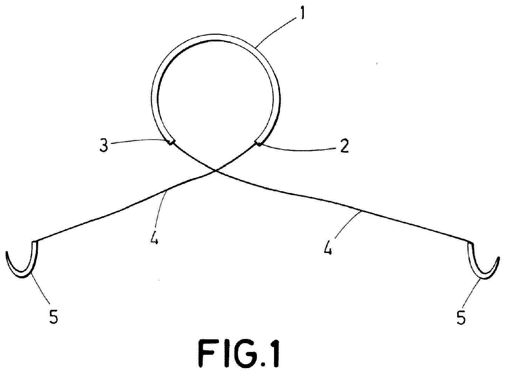

Figure 1. A plan view of the subaortic ring prior to implantation, showing the main constitutive components.

### PREFERRED EMBODIMENT OF THE INVENTION

Below is a detailed explanation, with the help of the previously mentioned figure, of a preferred embodiment of the object of this invention.

The resorbable subaortic ring described comprises an open circular segment (1), made of a resorbable, preferably flexible material such as a polydioxanone polymer, which when surgically implanted performs the annuloplasty or reduction of the dilated native ring to its natural size. Said open circular segment (1) has an essentially cylindrical and solid structure, preferably with a diameter of 1.5 millimetres.

Two suture threads (4) emerge from both a first end (2) and from a second end (3) of this open circular segment (1), made of a polypropylene and preferably a monofilament of calibre 2/0, to attach to the ring of the diseased heart. These suture threads (4) are linked to the first end (2) and the second end (3) of the open circular segment seamlessly by welding. The free ends of the suture threads (4) comprise a pair of semi-circular needles (5) to enable linking the suture threads (4) with the native tissues by sewing.

The final shape of the subaortic ring is achieved in the last step of its surgical implantation, the time when the semi-circular needles (5) are pulled away from the free ends of the suture threads (4) to tie them together, obtaining a closed ring with the desired diameter, which is preferably 26 millimetres or 28 millimetres, depending on the dilation of the native ring.

## Claims

1. Resorbable subaortic ring, specially designed to be implanted in subaortic annuloplasty operations, **characterised in that** it comprises:
- an open circular segment (1)with a first end (2) and a second end (3), the segment (1) further comprising a solid cylindrical structure, and
- a pair of suture threads (4) emerging the first end (2) and the second end (3) of the open circular segment (1), wherein threads (4) comprise polypropylene monofilaments and terminate in a pair of semi-circular needles (5).

2. Resorbable subaortic ring of claim 1 wherein the open circular segment (1) is made of flexible and resorbable material.

3. Resorbable subaortic ring of claim 2 wherein the flexible and resorbable material of the open circular segment (1) comprises a polydioxanone polymer.

4. Resorbable subaortic ring of claim 1 wherein the polypropylene monofilaments of the suture threads (4) are of calibre 2/0.

## Patentansprüche

1. Resorbierbarer subaortischer Ring, besonders ausgelegt, um bei subaortischen Annuloplastie-Eingriffen implantiert zu werden, **dadurch gekennzeichnet, dass** er Folgendes umfasst:
- ein offenes Kreissegment (1) mit einem ersten Ende (2) und einem zweiten Ende (3), wobei das Segment (1) ferner eine feste zylindrische Struktur umfasst und
- ein Paar Nahtfäden (4), die aus dem ersten Ende (2) und dem zweiten Ende (3) des offenen Kreissegments (1) herauskommen, wobei die Fäden (4) Polypropylen-Monofilamente umfassen und in einem Paar halbkreisförmiger Nadeln (5) enden.

2. Resorbierbarer subaortischer Ring nach Anspruch 1, wobei das offene Kreissegment (1) aus einem flexiblen und resorbierbaren Material hergestellt ist.

3. Resorbierbarer subaortischer Ring nach Anspruch 2, wobei das flexible und resorbierbare Material des offenen Kreissegments (1) ein Polydioxanon-Polymer umfasst.

4. Resorbierbarer subaortischer Ring nach Anspruch 1, wobei die Polypropylen-Monofilamente der Nahtfäden (4) der Stärke 2/0 sind.

## Revendications

1. Anneau sous-aortique résorbable, particulièrement conçu pour être implanté dans des interventions d'annuloplastie sous-aortique, **caractérisé en ce qu'**il comprend :
- un segment circulaire ouvert (1) avec une première extrémité (2) et une deuxième extrémité (3), le segment (1) comprenant en outre une structure cylindrique solide, et
- une paire de fils de suture (4) émergeant de la première extrémité (2) et de la deuxième extrémité (3) du segment circulaire ouvert (1), dans lequel les fils (4) comprennent des monofilaments de polypropylène et terminent en une paire d'aiguilles semi-circulaires (5).

2. Anneau sous-aortique résorbable selon la revendication 1 dans lequel le segment circulaire ouvert (1) est fait en un matériau flexible et résorbable.

3. Anneau sous-aortique résorbable selon la revendication 2 dans lequel le matériau flexible et résorbable du segment circulaire ouvert (1) comprend un polymère polydioxanone.

4. Anneau sous-aortique résorbable selon la revendication 1 dans lequel les monofilaments de polypropylène des fils de suture (4) sont de calibre 2/0.
